# EUROPEAN PATENT APPLICATION

(11) **EP 4 201 341 A1**
(43) Date of publication of application: **28.06.2023**
(21) Application number: 21290095.5
(22) Date of filing: 21.12.2021
(51) Int. Cl.: A61B 8/08

(54) **AORTIC ABDOMINAL ANEURYSM BIOMARKERS**

(71) Applicant: Koninklijke Philips N.V., 5656 AG Eindhoven (NL)
(72) Inventor: Germond Rouet, Laurence, 5656 AE Eindhoven (NL); Morales Varela, Hernan Guillermo, 5656 AE Eindhoven (NL); Antonuccio, Maria Nicole, 5656 AE Eindhoven (NL); This, Alexandre, 5656 AE Eindhoven (NL)
(74) Representative: Philips Intellectual Property & Standards

(57) **Abstract**

A method and system are provided for determining hemodynamic biomarkers of aortic abdominal aneurysms. A geometry of the aorta is obtained and at least three ultrasound Doppler planes are obtained. One of the Doppler planes captures the aorta's main axis and the other two Doppler planes capture two different cross sections of the aorta. A three dimensional, 3D, velocity field within the aorta is then derived, as well as a 3D relative pressure field within the aorta, using the at least three ultrasound Doppler planes and the aorta geometry. A reference pressure measurement is obtained from the subject and a 3D absolute pressure field within the aorta can then be obtained based on the relative pressure field and the reference pressure measurement to function as a biomarker for aortic abdominal aneurysms.

## Description

### FIELD OF THE INVENTION

The invention relates to the field of aortic abdominal aneurysms. In particular, the invention relates to biomarkers for aortic abdominal aneurysms.

### BACKGROUND OF THE INVENTION

An aortic abdominal aneurysm (AAA) is a localized enlargement of the infrarenal aorta of more than 50% of the normal vessel diameter. AAA is associated with a remarkable risk of morbidity and mortality and accounts for more than 175,000 deaths globally every year. AAA rupture is the most catastrophic complication associated with an AAA and typically leads to 80% mortality rate.

Current clinical guidelines base the AAA risk of rupture on morphological features such as the maximum diameter and the growth rate. An aneurysm diameter greater or equal to 5.5 cm or a growth rate greater of 1 cm/year are generally accepted as indication for repair. Once the patient is referred to the surgeon, computed tomography (CT) imaging is generally used to assess the diameter as CT has a relatively high spatial resolution.

However, CT imaging has high installation and maintenance costs, and ionizing radiation is released during the image acquisitions. Moreover, CT lacks both temporal information (unless gated used with an electrocardiogram) and flow information (unless complemented with computational fluid dynamic (CFD) simulations). In the absence of patient functional flow data, the added CFD simulation assumes the hemodynamic condition of the patient. However, CFD is typically a complex and slow process.

Thus, CT imaging generally makes CT unfeasible for early diagnosis and patient monitoring.

A problem also arises in that many AAAs can rupture with a diameter of less than 5.5 cm while larger ones might not rupture until much later (or not rupture). For this reason, a morphological assessment is broadly debated, and progress is needed toward better biomarkers. These biomarkers should be more sensitive than diameter and growth rate to guide clinical decision making, especially in asymptomatic patients.

Biophysics can be used to provide those biomarkers. Indeed, it is known that hemodynamics plays a crucial role in the initiation, development, and potential AAA rupture. On one hand, blood flow patterns from a 3D velocity field are associated with shear forces, fluid residence time and thrombus formation within an AAA, which are linked to the arterial wall degeneration and weakening. On the other hand, the local blood pressure is responsible for wall rupture.

Flow patterns can be captured by magnetic resonance imaging (MRI) and, partially, with 2D ultrasound (US) imaging. Nevertheless, part of the information describing the flow dynamics, such as the pressure field, is still missing.

In general, existing acquisition practices do not provide the necessary information to extract AAA biomarkers. In the case of MRI, both morphological and functional information are available, but MRI is very expensive compared to US. MRI is not portable and very difficult to democratize. Thus, MRI is mainly reserved for subjects where the diagnosis is ambiguous.

In the case of US imaging, current clinical protocols using 2D color Doppler imaging are not capable to properly capture the rotation of the flow (out of plane secondary flow), and therefore, a full 3D flow characterization is still missing. Moreover, US provides a limited field of view, making difficult to retrieve the morphological features (mainly the abdominal aortic neck or the iliac bifurcation) that leads to the rotational flow feature in the abdominal aorta.

Thus, there is a need for an improved and more accessible method for determining AAA biomarkers.

### SUMMARY OF THE INVENTION

The invention is defined by the claims.

According to examples in accordance with an aspect of the invention, there is provided a method for determining hemodynamic biomarkers of aortic abdominal aneurysms, the method comprising:
obtaining a geometry of the aorta in the abdominal region of a subject;
obtaining at least three ultrasound Doppler planes of the aorta in the abdominal region of the subject, wherein:
   one of the Doppler planes captures the aorta's main axis; and
   the other two Doppler planes capture two different cross sections of the aorta;
deriving a three dimensional, 3D, velocity field within the aorta and deriving a 3D relative pressure field within the aorta using the three ultrasound Doppler planes and the aorta geometry;
obtaining a reference pressure measurement from the subject; and
deriving a 3D absolute pressure field within the aorta based on the relative pressure field and the reference pressure measurement to function as a biomarker for aortic abdominal aneurysms.

Ultrasound imaging addresses the issues explained above relating to the use of MRI or CT imaging, as the devices are typically cheaper and more accessible. However, ultrasound imaging typically only captures 2D flow (i.e. Doppler) and only provides limited information due to its limited field of view.

Thus, it is proposed to derive flow information (3D velocity and pressure fields) in the aorta from three ultrasound Doppler planes. The three Doppler planes capture three different planes of the aorta and thus they provide information of the flow of blood in the aorta. As the aorta has a tubular geometry, it can be accurately assumed that the flow pattern will likely be dominated by fluid flow inertia and geometry and thus a relatively smooth field can be assumed. As a smooth field can be assumed, the three Doppler planes provide enough information to reconstruct the flow information in the aorta accurately.

The flow information (i.e. 3D velocity and 3D pressure fields) can thus be used as biomarkers for AAA and to inform a clinician on the risk of rupture of the aneurysm.

The reference pressure measurement may be obtained using cuff pressure measurements. Other pressure measurement of the subject methods may also be used.

Deriving the 3D velocity field and the 3D relative pressure field for example comprises:
deriving two dimensional, 2D, velocity fields relative to the aorta geometry for each of the Doppler planes;
reconstructing the 3D velocity field within the aorta by interpolating the velocity between the 2D velocity fields; and
deriving the 3D relative pressure field from the 3D velocity field.

This provides a method for reconstructing the flow information with relatively small processing requirements. The aorta geometry, in essence, delimitates the fluid-domain defining the physic boundary of the flow. Thus, from the 2D velocity fields, a 3D velocity field can be reconstructed within the boundaries of the aorta geometry.

Deriving the 3D velocity field and the 3D relative pressure field may instead comprise:
performing a computational fluid dynamic, CFD, simulation of the blood flow through the aorta by:
   using the Doppler planes to generate reference velocity values; and
   using the aorta geometry to define the computational domain,
wherein the CFD simulation is configured to output the 3D velocity field and the 3D relative pressure field.

This provides a method for reconstructing the flow information with higher accuracy and precision. The Doppler planes provide input reference velocity values and the aorta geometry provides the computational domain for the CFD. This information can be input into the generation of the CFD to generate an accurate simulation of the blood flow in the aorta of the subject.

Obtaining the aorta geometry for example comprises obtaining an ultrasound volume of the aorta of the subject and segmenting the geometry of the aorta from the ultrasound volume.

Using ultrasound volume imaging for obtaining the aorta geometry enables the geometry and the Doppler planes to be acquired and visualized using ultrasound. Thus, they could be acquired using the same ultrasound probe, thereby improving the efficiency of obtaining the aorta geometry and the Doppler planes.

Additionally, if they are acquired with the same probe, it may enable the Doppler planes to be localized, relative to the aorta geometry, much faster as the user may not move the probe during the acquisitions, or the movement of the probe could be obtained.

Obtaining the three Doppler planes for example comprises obtaining one or more Doppler cine-loops, wherein at least one plane of the one or more Doppler cine-loops corresponds to the aorta's main axis and at least two planes correspond to different cross sections of the aorta.

Doppler cine-loops further provide temporal information of the flow of blood in the aorta, thus improving the accuracy of the derived flow information.

The method may further comprise registering the at least three Doppler planes and the aorta geometry to a common coordinate system.

The method may further comprise generating a risk of clot by identifying regions of the aorta geometry wherein the corresponding region of the 3D velocity field indicates a velocity lower than a velocity threshold.

Clots can damage the wall of the aorta which increases the risk of AAA rupture. Thus, an indication of clot (i.e. whether there may be a clot or not) can provide information on how likely an AAA is to rupture and whether it could rupture prematurely (i.e. at smaller diameters than usual).

The method for example further comprises generating a risk of rupture by identifying regions of the aorta geometry where the corresponding 3D absolute pressure field indicates a pressure higher than a pressure threshold.

High pressures in the aorta can be an indication that an AAA is likely to rupture.

The invention also provides a computer program comprising computer program code which is adapted, when said program is run on a computer, to implement the method as defined above.

The invention also provides a system for determining hemodynamic biomarkers of aortic abdominal aneurysms, comprising:
a 3D ultrasound imaging unit; and
a processor for processing received ultrasound images from the 3D ultrasound imaging unit,
wherein the processor is adapted to operate the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a method for obtaining biomarkers for AAAs;
Figure 2 illustrates the acquisition of ultrasound Doppler planes of the aorta;
Figure 3 shows a color Doppler biplane acquisition of the aorta;
Figure 4 shows an exemplary method for obtaining a 3D absolute pressure field reconstruction of an AAA;
Figure 5 illustrates a CFD simulation of the blood flow in the abdominal aorta;
Figure 6 show the results of the feasibility test for the reconstruction algorithm; and
Figure 7 shows a system for obtaining biomarkers for AAAs.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method and system for determining hemodynamic biomarkers of aortic abdominal aneurysms. A geometry of the aorta is obtained and three ultrasound Doppler planes are obtained. One of the Doppler planes captures the aorta's main axis and the other two Doppler planes capture two different cross sections of the aorta. The aorta's main axis may often be referred to as the "longitudinal axis" in the art. A three dimensional, 3D, velocity field within the aorta is then derived, as well as a 3D relative pressure field within the aorta, using the three ultrasound Doppler planes and the aorta geometry. A 3D absolute pressure field within the aorta can then be obtained based on the relative pressure field and a reference pressure measurement (e.g. branchial pressure measurement), to function as a biomarker for aortic abdominal aneurysms.

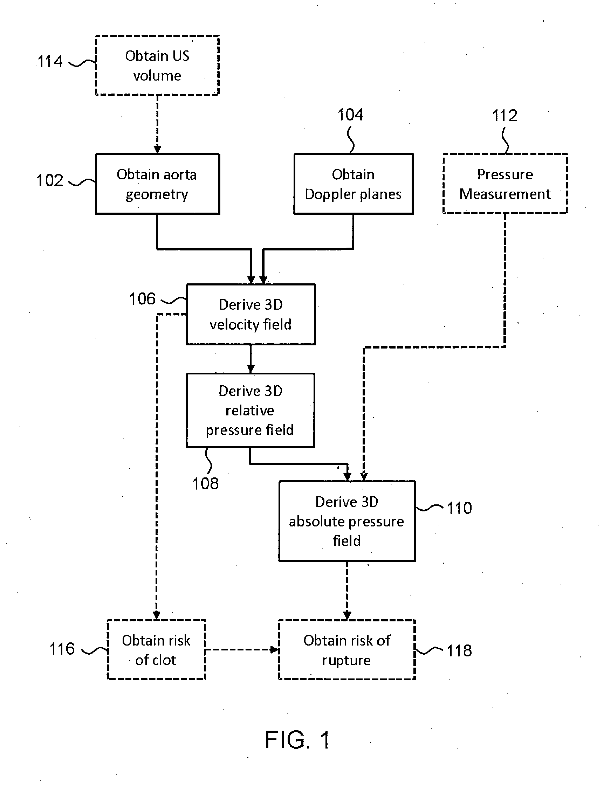

Figure 1 shows the basic steps of a method for obtaining biomarkers for aortic abdominal aneurysms, AAAs, in particular in the form of a 3D velocity field and a 3D absolute pressure field which function as hemodynamic biomarkers. The method comprises obtaining an aorta geometry in step 102 and obtaining at least three Doppler planes in step 104. One of the Doppler planes crosses the aorta along the main axis whilst the other two Doppler planes cross the aorta at different cross sections of the aorta.

A 3D velocity field can be derived in step 106 from the aorta geometry and the three Doppler planes. A 3D relative pressure field can thus be derived from the 3D velocity field in step 108. The 3D relative pressure field shows differences in pressure in the aorta but not the absolute local values of the pressure. Thus, a 3D absolute pressure field is derived from the 3D relative pressure field in step 110.

The 3D absolute pressure field may be derived by calibrating the 3D relative pressure field with a pressure measurement such as branchial cuff pressure measurements of the subject in step 112. Alternatively, other methods for calibrating the pressure may be used. These methods may be adapted from known methods of calibrating the central blood pressure. Thus the pressure measurement step 112 is optional.

Additionally, an ultrasound (US) volume of the aorta may be obtained in step 114. The US aorta volume can thus be segmented in order to obtain the aorta geometry. Thus, both the aorta geometry and Doppler planes can both be obtained using ultrasound.

The velocity field may be used to obtain a risk of clot in step 116. Additionally, the absolute pressure field and/or the risk of clot may be used to obtain a risk of rupture for the AAA in step 118.

In summary, it is proposed to perform specific US acquisitions and combine them with biophysics techniques and numerical techniques to obtain a full flow characterization of AAA hemodynamics.

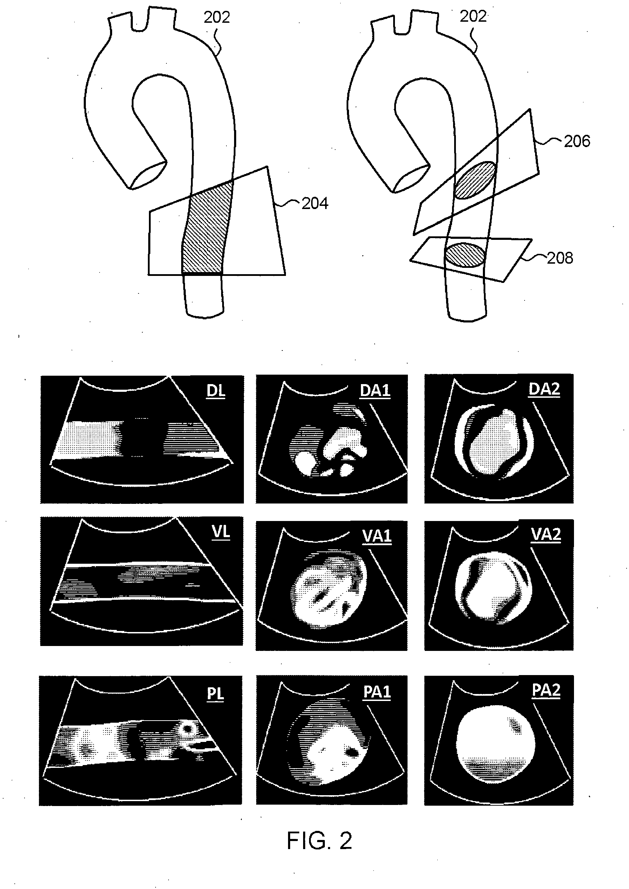

Figure 2 illustrates the acquisition of ultrasound Doppler planes of the aorta. An aorta 202 is illustrated with three planes passing through the aorta. The planes may be obtained via biplane color Doppler image cine-loop acquisition.

At least three planes, one longitudinal and two cross-sections, are required. Depending on the probe being used, these planes can be obtained with one, two or three cine-loops. If more than one cine-loop is acquired, they may need to be temporally registered to the other cine-loops.

If several cross-sectional planes are acquired, the flow reconstruction may be more precise. In this case, three planes are acquired. Figure 2 shows plane 204 that captures the arterial main axis, plane 206 that captures a first arterial cross-section and plane 208 that captures a second arterial cross-sections.

Image DL in Figure 2 is a Doppler plane corresponding to plane 204, image DA1 is a Doppler plane corresponding to plane 206 and image DA2 is a Doppler plane corresponding to plane 208. The velocity and pressure field reconstructions related to DL, DA1, and DA2 are also shown. Image VL is a 2D velocity reconstruction from image DL, image VA1 is a 2D velocity reconstruction from image DA1 and image VA2 is a 2D velocity reconstruction from image DA2.

Similarly, image PL is a 2D pressure reconstruction from image VL, image PA1 is a 2D pressure reconstruction from image VA1 and image PA2 is a 2D pressure reconstruction from image VA2.

As can be seen from the images DL, VL and PL, the flow of blood in the abdominal aorta is fairly smooth.

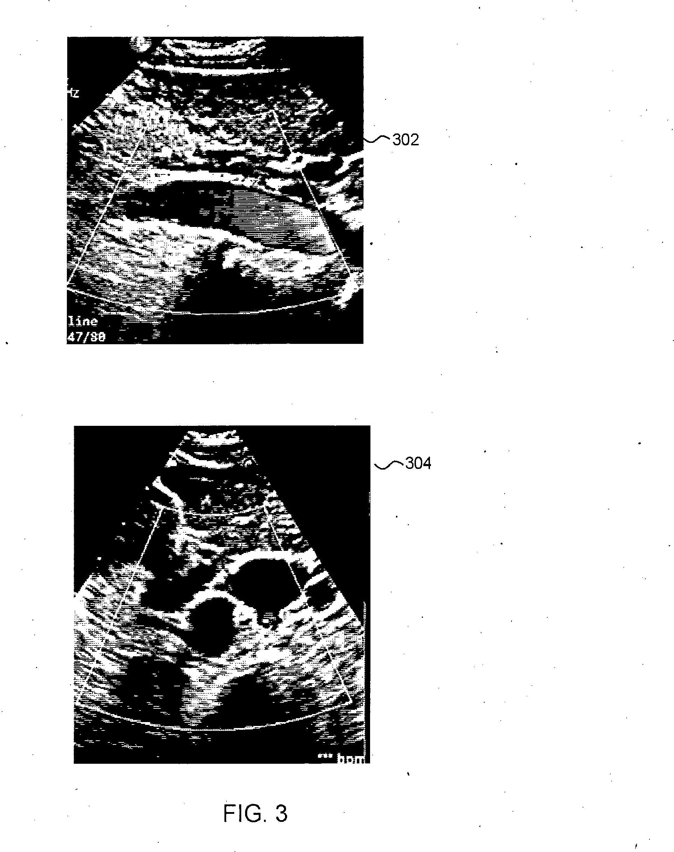

Figure 3 shows a color Doppler biplane acquisition of the aorta. Image 302 is a cine-loop frame which shows a color Doppler acquisition of the main arterial axis and image 304 is the same cine-loop frame which shows a color Doppler acquisition of a cross section of the aorta.

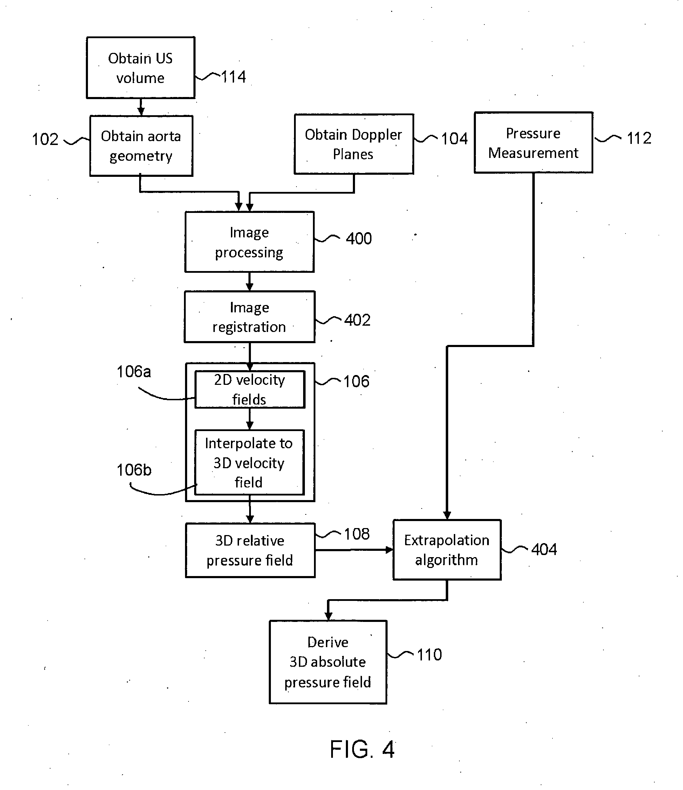

Figure 4 is used to explain in more detail one particular more detailed implementation of the steps of Figure 1 for deriving a 3D absolute pressure field and a 3D velocity field. The 3D velocity field and 3D absolute pressure field may be used to obtain a risk of clot and a risk of rupture in the same way as explained with reference to Figure 1. However, these steps are not repeated in Figure 4.

In this case, 3D US B-mode acquisitions in step 114 are used to obtain the aorta geometry in step 102 as above, and are combined with dynamic Doppler image cine-loops obtained in step 104 to extract both velocity and pressure fields of the AAA.

In order to obtain the 3D US B-mode acquisitions in step 114, a 3D matrix probe may be used. For example a Phillips X6-1 xMatrix probe may be used. The probe is positioned on the subject in such a way that a volumetric image of the AAA and the parent artery can be acquired.

After the volumetric image is obtained in step 114, the probe (or a different probe) acquires the color-Doppler cine-loops in step 104. One of the cine-loop planes is acquired along the vessel main axis and at least one other cine-loop plane should be perpendicular (or close to perpendicular) to the main flow direction in order to retrieve the flow rotation.

A cine-loop is a collection of several color Doppler images. If a cine-loop is available, then the temporal variation of that velocity field can be added. The velocity can be reconstructed from either a single US Doppler image or cine-loops.

Both the volumetric image obtained in step 114 and the cine-loops obtained in step 104 are passed through an image processing algorithm 400. For example, they may both be corrected (e.g. de-noised). An algorithm can be applied during these steps to retrieve a segmentation of the AAA wall from the volumetric image.

The cine-loops obtained in step 104 are registered to the volumetric image obtained in step 114 by an image registration algorithm in step 402 in a common coordinate system (in the case where the probe was moved during acquisitions). If the probe is not moved while acquiring both the volumetric image and the cine-loops, the image registration between these image acquisitions can be significantly improved.

A reconstruction algorithm is employed to derive a 2D velocity field within each cine-loop plane in step 106a as well as inside sections of the AAA volume which lack color Doppler data. In essence, the reconstruction algorithm derives the 3D velocity field in step 106 by using the color Doppler data from the cine-loops obtained in step 104.

The missing velocity components of the Doppler planes may be retrieved in step 106a thereby creating the 2D velocity vector field. With the calculated 2D velocity fields and the known 3D wall geometry (where the velocity is almost zero), the 3D velocity field can be interpolated in step 106b in the areas of the 3D volume where there is no Doppler data. Thus, deriving a 3D velocity field in step 106b involves interpolating 2D velocity fields to derive the 3D velocity field.

The reconstruction algorithm used in step 106 can be applied to each Doppler plane independently. Afterwards, obtaining a 2D velocity field of the Doppler planes with respect to the 3D volume, the same reconstruction algorithm can be applied to retrieve the rest of the velocities within the 3D volume.

Flow patterns in tubular structures are strongly driven by fluid flow inertia and geometry and, thus, they can be assumed to be smooth. In other words, with the partial information that can be retrieved from the Doppler planes and 3D volume, it is possible to obtain a good estimation of the velocities inside the abdominal aorta. This does not extend to more complex anatomies (e.g. the heart) where the non-linear mechanics of the system (e.g. ventricular motion, valve opening/close etc.) make this assumption unfeasible.

In this case, the velocity reconstruction algorithm optimizes a cost function that includes four terms. A first term is the deviation of the reconstructed velocity field with respect to the color Doppler data from the cine-loops obtained in step 104, a second term is the conservation of mass, a third term is the assumption of a smooth field and the fourth term includes specific constraints based on the biophysical condition of the problem.

The fourth term should be tailored to the specific biophysical problem. In the case of AAAs, blood flow at the inlet and outlet is mainly directed along the arterial axis, with a no-slip boundary conditions on the arterial wall.

Details of an exemplary reconstruction algorithm can be found in K. C. Assi "Intraventricular vector flow mapping - a Doppler-based regularized problem with automatic model selection," Physics in Medicine & Biology, vol. 62, p. 7131-7147, 2017. This paper describes the reconstruction of velocity fields from Doppler images for the left ventricle. However, the flow of blood in the left ventricle is fairly complex and thus difficult to reconstruct. It has been realized that the flow of blood in the abdominal aorta is less complex due to its geometry and inertia of the blood and, thus, the reconstruction algorithm will provide accurate results.

Additional cross-section cine-loops could be further acquired in step 104 to enrich the reconstruction of the 3D velocity field derived in step 106. Once the 3D velocity field is reconstructed, a relative 3D pressure field can be derived in step 108 by solving the Poisson equation or any other method that is known in the field to derive relative pressure fields from velocity fields. In order to obtain the 3D absolute pressure field in step 110 within the region of interest, an extrapolation algorithm can be used in step 404 on the 3D relative pressure field using a branchial pressure obtained in step 112 from, for example, cuff manometer measurements. The extrapolation can be performed with a 0D or 1D model of the arterial tree.

An exemplary extrapolation algorithm for the extrapolation step 404 can be found in P. Reymond "Validation of a one-dimensional model of the systemic arterial tree," American Journal of Physiology-Heart and Circulatory Physiology, vol. 297, no. 1, pp. 208-222, 2009.

Arteries are considered as straight long tapered segments with a viscoelastic wall. A one-dimensional (ID) model of the arterial tree can be obtained by integrating the continuity and longitudinal momentum equations of the Navier-Stokes equations. The obtained equations contain three primary variables: the volume flow rate (Q), the transmural pressure (P), and the instantaneous arterial lumen area (A). To solve the problem, one more equation is introduced in which the distending pressure P is related to the cross-sectional area A by considering the viscoelastic properties of the arterial wall. The cuff-pressure measurements are then employed in the model to predict the pressure in the desired vascular portion.

An alternative step to the reconstruction of the 3D velocity field and 3D relative pressure field may be to perform CFD simulations of the blood flow in the aorta. The CFD simulation may use the volumetric image obtained in step 114 to define the computational domain and the cine-loops as reference velocity data for the CFD simulations.

CFD solves the Navier-Stokes equations to find the velocity and pressure field inside a confined system. To well pose the problem, CFD requires boundary conditions.

In this case, the confined system is limited by the wall of the segmented 3D volume, as well as the Doppler planes with flow information. Additionally, CFD simulations may need to be complemented with particular assumptions at the inlet/outlet such as a parabolic profile.

In general, this method aims to use 2D Doppler US in combination with 3D US to build hemodynamics biomarkers. AAAs are monitored through US imaging, which is a portable, cheaper and more accessible modality than CT and MRI. Although US offers a limited field if view compared to the other modalities, it has the potential to provide sufficient temporal information.

In this case, a processor is used to apply the various algorithms. For example, an image registration algorithm is used for image registration between the 3D volume and cine-loops in step 402, an image processing algorithm is used for image de-aliasing and de-noising in step 400 and a reconstruction algorithm is used for 3D velocity field reconstruction in step 106. The reconstruction algorithm may also derive the 3D relative pressure field. An extrapolation algorithm is also used to provide an absolute local pressure in the region of interest.

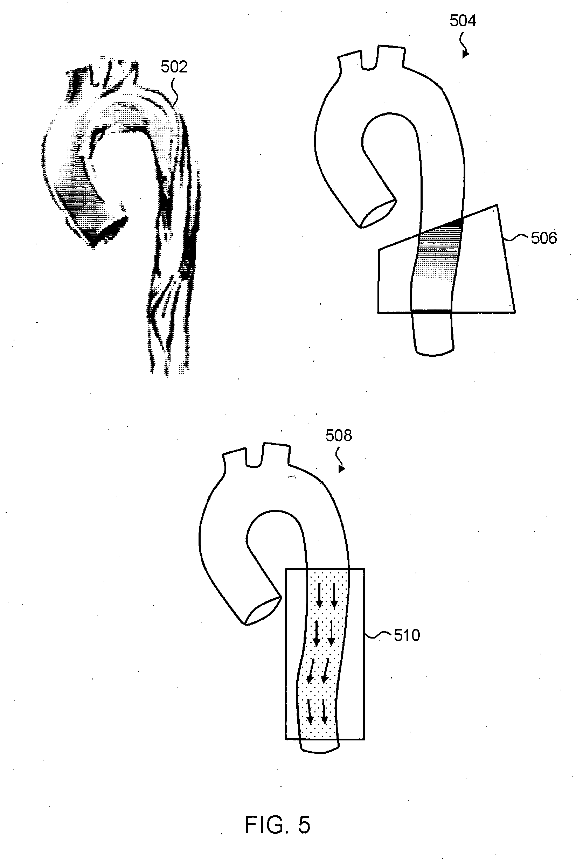

Figure 5 illustrates a CFD simulation of the blood flow in the abdominal aorta. Image 502 shows the resulting 3D velocity field from a CFD simulation. Although CFD simulations typically take longer and require more processing resources, they can be used for obtaining precise hemodynamic biomarkers (i.e. 3D velocity and pressure fields). Thus, a CFD simulation may be used to show the feasibility of using the reconstruction algorithm.

A CFD simulation was performed using the geometry of a healthy aorta and outputting a reconstructed 3D velocity field. The results are shown in image 502. The CFD simulation were performed on an image-based aorta including the aortic arc to induce the flow rotation.

A Doppler-like image generator was applied to the CFD simulation to obtain Doppler planes to input into the reconstruction algorithm. The Doppler-like image is used to simulate the acquisition of Doppler planes in the CFD simulation. This is shown in illustration 504. Only the Doppler plane 506 along the main axis of the abdominal aorta is shown as being acquired in illustration 504. However, at least two more Doppler planes are also acquired at two more cross sections of the abdominal aorta.

The Doppler-images generated from the CFD simulation were then input into the reconstruction algorithm to test the feasibility of the reconstruction algorithm. Illustration 508 illustrates the output 3D velocity field 510 obtained from the reconstruction algorithm.

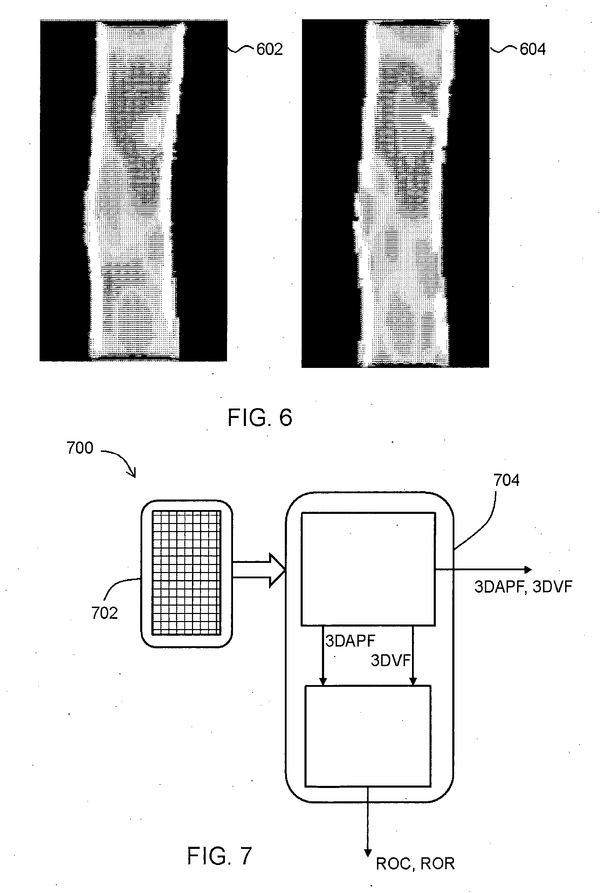

Figure 6 show the results of the feasibility test for the reconstruction algorithm. Image 602 shows a 2D slice of the ground truth data obtained from the CFD simulation and image 604 shows a corresponding 2D slice of the reconstructed 2D velocity field. The ground truth data is used for error quantification.

In other words, the reconstructed velocity from image 604 is compared against the original CFD velocity shown in image 602. The maximum errors in the velocity magnitude were around 15% and the maximum angle deviation was near 4%. Visual inspection of the images 602 and 604 shows that the reconstructed velocity field from image 604 is close to the ground truth velocity shown in image 602. Thus, the reconstruction algorithm provides a relatively accurate reconstruction of the velocity field in that plane.

From the 3D velocity and pressure fields, further analysis can be performed. A clot may occur in regions of the abdominal aorta where low velocity and high residence time are quantified. The arterial wall near that clot will be damaged and weakened due to the degenerative process caused by the clot. Those regions are potential spots for rupture and their identification (position and size) is thus of importance.

This can be a key discriminant to the large AAA where rupture will not happen. In other words, if a large AAA does not have a clot, or will not develop a clot, then it should be safer than an AAA where a clot is observed.

In the case of the 3D pressure field, regions with relative high pressure will have higher stresses associated to hemodynamics. This can be a key indicator for small AAA that do rupture since flow will remain relatively straight along the vessel axis and pressure values will be more important due to hemodynamics rather than due to morphological aspects (e.g. high curvature). A large population-based study could be conducted to determine the range where these biomarkers provide a safe condition for a given AAA.

The hemodynamic biomarkers may include the 3D velocity field, the 3D relative pressure field and the 3D absolute pressure fields. The risk of clot and the risk of rupture may also be biomarkers.

The geometry of the aorta is a 3D geometry of the aorta. The aorta's main axis is not meant to be limited as a single axis but as an axis in a possible range of axes which are aligned with the direction of the flow of blood. It will be understood that having the velocity data (from Doppler planes) in three different planes, one of which is perpendicular (or mostly perpendicular) to the rest, gives information on the 3D velocity.

Deriving the 3D velocity field may involve using a smooth field assumption for the 3D velocity field. A smooth field is a field which is at least differentiable everywhere on the field.

Figure 7 shows a system 700 for determining hemodynamic biomarkers of aortic abdominal aneurysms. The system comprises a 3D ultrasound imaging unit 702 and a processor 704 for processing received ultrasound images from the 3D ultrasound imaging unit. The processor performs the steps as explained above to derive a three dimensional, 3D, velocity field (3DVF) and a 3D absolute pressure field (3DAPF) within the aorta based on a relative pressure field and the reference pressure measurement. The velocity field and pressure field function as a biomarker for aortic abdominal aneurysms.

The processor 704 may also derive a risk of clot (ROC) and a risk of rupture (ROR).

The skilled person would be readily capable of developing a processor for carrying out any herein described method. Thus, each step of a flow chart may represent a different action performed by a processor, and may be performed by a respective module of the processor.

As discussed above, the system makes use of a processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A method for determining hemodynamic biomarkers of aortic abdominal aneurysms, the method comprising:
(102) obtaining a geometry of the aorta in the abdominal region of a subject;
(104) obtaining at least three ultrasound Doppler planes of the aorta in the abdominal region of the subject, wherein:
one of the Doppler planes captures the aorta's main axis; and
the other two Doppler planes capture two different cross sections of the aorta;
(106) deriving a three dimensional, 3D, velocity field within the aorta and (110) deriving a 3D relative pressure field within the aorta using the at least three ultrasound Doppler planes and the aorta geometry;
(106) obtaining a reference pressure measurement of the subject; and
(110) deriving a 3D absolute pressure field within the aorta based on the relative pressure field and the reference pressure measurement to function as a biomarker for aortic abdominal aneurysms.

2. The method of claim 1, wherein deriving the 3D velocity field and deriving the 3D relative pressure field comprises:
(106a) deriving two dimensional, 2D, velocity fields relative to the aorta geometry for each of the Doppler planes;
(106b) reconstructing the 3D velocity field within the aorta by interpolating the velocity between the 2D velocity fields; and
(108) deriving the 3D relative pressure field from the 3D velocity field.

3. The method of claim 1, wherein deriving the 3D velocity field and the 3D relative pressure field comprises:
performing a computational fluid dynamic, CFD, simulation of the blood flow through the aorta by:
using the Doppler planes to generate reference velocity values; and
using the aorta geometry to define the computational domain,
wherein the CFD simulation is configured to output the 3D velocity field and the 3D relative pressure field.

4. The method of any one of claims 1 to 3, wherein (102) obtaining the aorta geometry comprises (114) obtaining an ultrasound volume of the aorta of the subject and segmenting the geometry of the aorta from the ultrasound volume.

5. The method of any one of claims 1 to 4, wherein (104) obtaining the at least three Doppler planes comprises obtaining one or more Doppler cine-loops, wherein at least one frame of the one or more Doppler cine-loops corresponds to the aorta's main axis and at least two frames correspond to different cross sections of the aorta.

6. The method of any one of claims 1 to 5, further comprising (116) generating a risk of clot by identifying regions of the aorta geometry wherein the corresponding region of the 3D velocity field indicates a velocity lower than a velocity threshold.

7. The method of any one of claims 1 to 6, further comprising (118) generating a risk of rupture by identifying regions of the aorta geometry where the corresponding 3D absolute pressure field indicates a pressure higher than a pressure threshold.

8. A computer program comprising computer program code which is adapted, when said program is run on a computer, to implement the method of any one of claims 1 to 7.

9. A system (700) for determining hemodynamic biomarkers of aortic abdominal aneurysms, comprising:
a 3D ultrasound imaging unit (702); and
a processor (704) for processing received ultrasound images from the 3D ultrasound imaging unit, wherein the processor is adapted to:
obtain a geometry of the aorta in the abdominal region of a subject;
obtain at least three ultrasound Doppler planes of the aorta in the abdominal region of the subject, wherein:
one of the Doppler planes captures the aorta's main axis; and
the other two Doppler planes capture two different cross sections of the aorta;
derive a three dimensional, 3D, velocity field within the aorta and a 3D relative pressure field within the aorta using the at least three ultrasound Doppler planes and the aorta geometry;
obtain a reference pressure measurement of the subject; and
derive a 3D absolute pressure field within the aorta based on the relative pressure field and the reference pressure measurement to function as a biomarker for aortic abdominal aneurysms.

10. The system of claim 9, wherein the processor (704) is adapted to derive the 3D velocity field and the 3D relative pressure field by:
deriving two dimensional, 2D, velocity fields relative to the aorta geometry for each of the Doppler planes;
reconstructing the 3D velocity field within the aorta by interpolating the velocity between the 2D velocity fields; and
deriving the 3D relative pressure field from the 3D velocity field,

11. The system of claim 9, wherein the processor (704) is adapted to derive the 3D velocity field and the 3D relative pressure field by:
generating a computational fluid dynamic, CFD, simulation of the blood flow through the aorta by:
using the Doppler planes to generate reference velocity values; and
using the aorta geometry to define the computational domain,
wherein the CFD simulation is configured to output the 3D velocity field and the 3D relative pressure field.

12. The system of any one of claims 9 to 11, wherein the processor (704) is adapted to obtain the aorta geometry by obtaining an ultrasound volume of the aorta of the subject and segmenting the geometry of the aorta from the ultrasound volume.

13. The system of any one of claims 9 to 12, wherein the processor (704) is adapted to obtain the at least three Doppler planes by obtaining one or more Doppler cine-loops, wherein at least one frame of the one or more Doppler cine-loops corresponds to the aorta's main axis and at least two frames correspond to different cross sections of the aorta.

14. The system of any one of claims 9 to 13, wherein the processor (704) is adapted generate a risk of clot by identifying regions of the aorta geometry wherein the corresponding region of the 3D velocity field indicates a velocity lower than a velocity threshold.

15. The system of any one of claims 9 to 14, wherein the processor (704) is adapted to generate a risk of rupture by identifying regions of the aorta geometry where the corresponding 3D absolute pressure field indicates a pressure higher than a pressure threshold.
